# EUROPÄISCHE PATENTSCHRIFT

(11) **EP 4 065 559 B1**
(45) Veröffentlichungstag und Bekanntmachung des Hinweises auf die Patenterteilung: **16.08.2023**
(21) Anmeldenummer: 20726865.7
(22) Anmeldetag: 26.05.2020
(51) Int. Cl.: C07D 211/58

(54) **VERBESSERTES VERFAHREN ZUR HERSTELLUNG VON 4-METHYLAMMONIUM-2,2,6,6-TETRAMETHYLPIPERIDINYLCHLORIDEN**
IMPROVED PROCESS FOR THE PREPARATION OF 4-METHYLAMMONIUM-2,2,6,6-TETRAMETHYLPIPERIDINYL CHLORIDES
PROCÉDÉ AMÉLIORÉ POUR LA PRÉPARATION DE CHLORURE DE 4-METHYLAMMONIUM-2,2,6,6-TETRAMETHYLPIPERIDINE

(30) Priorität: 29.11.2019 EP 19212524
(43) Veröffentlichungstag der Anmeldung: 05.10.2022
(73) Patentinhaber: Evonik Operations GmbH, 45128 Essen (DE)
(72) Erfinder: HEIDELBERG, Julia, 48143 Münster (DE); GÄRTNER, Felix, 45721 Haltern am See (DE)
(74) Vertreter: Evonik Patent Association
(86) Internationale Anmeldenummer: PCT/EP2020/064501
(87) Internationale Veröffentlichungsnummer: WO 2021/104685

(56) Entgegenhaltungen:
- WO-A1-2018/028830
- TOBIAS JANOSCHKA ET AL: "An Aqueous Redox-Flow Battery with High Capacity and Power: The TEMPTMA/MV System", ANGEWANDTE CHEMIE, INTERNATIONAL EDITION, Bd. 55, Nr. 46, 18. Oktober 2016 (2016-10-18), Seiten 14427-14430, XP055494176, DE ISSN: 1433-7851, DOI: 10.1002/anie.201606472 in der Anmeldung erwähnt

## Beschreibung

Die vorliegende Erfindung betrifft ein Verfahren zur Herstellung von 4-Methylammonium-2,2,6,6-tetramethylpiperidinylchloriden. In diesem Verfahren wird eine Schmelze der Edukte eingesetzt, und diese direkt mit dem Alkylierungsmittel Methylchlorid umgesetzt. Diese Vorgehensweise gewährleistet eine schnelle und effiziente Umsetzung. In einer bevorzugten Ausführungsform wird diese Reaktion kontinuierlich, zum Beispiel in einem Schlaufenreaktor, durchgeführt.

### Hintergrund der Erfindung

Die Synthese von 4-Ammonium-alkylpiperidin-1-yl-salzen ist von großer Bedeutung für das Gebiet der organischen Batterien, in welchen solche Verbindungen als Vorstufen zur Synthese von Elektrolytmaterial eingesetzt werden.

Die Synthese solcher Materialien ist vielfach im Stand der Technik beschrieben und erfolgt üblicherweise durch Umsetzung von 4-Aminoalkylpiperidin mit einem Alkylhalogenid zum 4-Ammonium-alkylpiperidinylsalz.

Als Alkylierungsmittel wird im Stand der Technik üblicherweise das entsprechende Alkyljodid eingesetzt, wie etwa von C. Francavilla et al., Bioorganic and Medicinal Chemistry Letters 2011, 21, 3029-3033 auf Seite 3031 in Schema 6 bei der Umsetzung von Verbindung **38** in **39** beschrieben. Die gleiche Reaktion wird auch in Janoschka et al., Angew. Chem. Int. Ed. 2016, 55, 14427-14430 (im Folgenden "Janoschka et *al*.") auf Seite 14429 in Schema 1 bei der Umsetzung von Verbindung **2** in **3** beschrieben.

B. Bales et al., J. Phys. Chem. A 2009, 113, 9295-9303 beschreiben auf Seite 9298, Schema 1, die Umsetzung eines deuterierten 4-Aminoalkylpiperidinderivats mit *n*-Dodecylbromid zum entsprechenden Bromidsalz.

B. K. Sinha & C. F. Chignell, J. Med. Chem. 1975, 18, 669-673 (im Folgenden "B. K. Sinha & C. F. Chignell") beschreiben die entsprechende Umsetzung von 4-Dimethylamino-2,2,6,6-tetramethylpiperidin mit 1,6-Dibromhexan und 1,10-Dibromdecan zum jeweiligen Bromidsalz.

Als Folgestufe zur Alkylierung wird typischerweise das erhaltene 4-Ammonium-alkylpiperidinylsalz mit einem Oxidationsmittel wie beispielsweise HzOz zum 4-Ammonium-alkylpiperidin-1-yloxysalz oxidiert.

Sowohl Janoschka et *al.* als auch B. K. Sinha & C. F. Chignell beschreiben diese weitere Stufe, die Oxidation der sekundären Aminogruppe zum jeweiligen N-O·-Radikal. Diese wird mit HzOz und Natriumwolframat durchgeführt.

Die im Stand der Technik beschriebenen Alkylierungsverfahren verlaufen demnach hauptsächlich durch Umsetzung mit Alkylbromid oder Alkyljodid. Dies ist insofern von Nachteil, als dass in den Fällen, in denen ein Chloridsalz gewünscht ist, ein Anionenaustausch des Jodids oder Bromids mit Chlorid erfolgen muss, was einen zusätzlichen Schritt erfordert und die gesamte Synthese somit aufwendiger macht.

Eine direkte Umsetzung des 4-Aminopiperidins mit Alkylchloriden und Sulfaten ist in der WO 2018/028830 A1 und von Janoschka et *al*. beschrieben. In diesen Offenbarungen erfolgt speziell eine direkte Umsetzung mit Methylchlorid. Der Einsatz von Methylchlorid hat zwar den Nachteil, dass Methylchlorid weniger reaktiv ist als das entsprechende Methyljodid oder Methylbromid und die Reaktion somit langsamer verläuft. Allerdings ermöglicht diese in WO 2018/028839 A1 beschriebene Reaktion zuverlässig einen direkten Zugang zum Chloridsalz.

Es besteht jedoch weiterhin das Bedürfnis nach verbesserten Verfahren zur Synthese von 4-Ammonium-alkylpiperidinylchloriden, welche sich durch eine einfache und effiziente Vorgehensweise auszeichnen. Sie sollen insbesondere einen effizienten Einsatz der Edukte gewährleisten. Es wurde nun ein Verfahren gefunden, welches diese Aufgabe löst.

### Detaillierte Beschreibung der Erfindung

Die vorliegende Erfindung betrifft demnach ein Verfahren zur Herstellung mindestens einer Verbindung der Formel **(I-A)** oder **(I-B),** bevorzugt Formel **(I-A),** mit 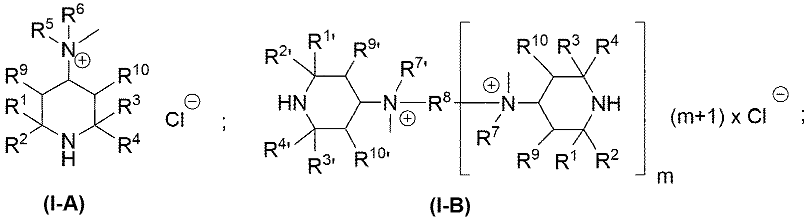
wobei R¹, R², R³, R⁴, R¹', R²', R³', R⁴' unabhängig voneinander jeweils ein C₁-C₆-Alkylrest, insbesondere ein C₁-C₄-Alkylrest, bevorzugt ein C₁-C₂-Alkylrest, und bevorzugter alle jeweils Methyl sind,
wobei R⁹, R¹⁰, R⁹', R¹⁰' unabhängig voneinander jeweils aus der Gruppe bestehend aus Wasserstoff, C₁-C₆-Alkylrest, insbesondere aus der Gruppe bestehend aus Wasserstoff, C₁-C₄-Alkylrest, bevorzugt aus der Gruppe bestehend aus Wasserstoff, Methyl, Ethyl ausgewählt sind, und bevorzugter alle Wasserstoff sind,
wobei R⁵, R⁶, R⁷, R⁷' unabhängig voneinander jeweils aus der Gruppe bestehend aus Wasserstoff, Alkyl, Cycloalkyl, Aryl, Aralkyl, Heterocyclyl ausgewählt sind, wobei die Reste Alkyl, Cycloalkyl, Aryl, Aralkyl, Heterocyclyl mit ein oder mehreren Resten ausgewählt aus der Gruppe bestehend aus Alkoxy, Hydroxy, Carboxyl, Carboxylester, Carboxylamid, Sulfonat, Sulfonsäureester, Sulfonamid, Halogen substituiert sein können, wobei außerdem Alkyl durch ein oder mehrere, nicht direkt zueinander benachbarte zweiwertige Reste ausgewählt aus der Gruppe bestehend aus Sauerstoff, Imino unterbrochen sein kann,
und wobei außerdem R⁵ und R⁶ zusammen eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können und
wobei insbesondere R⁵, R⁶, R⁷, R⁷' unabhängig voneinander jeweils aus der Gruppe bestehend aus Wasserstoff, Alkyl, wobei Alkyl durch ein oder mehrere, nicht direkt zueinander benachbarte zweiwertige Reste ausgewählt aus der Gruppe bestehend aus Sauerstoff, Imino unterbrochen sein kann, ausgewählt sind, und wobei außerdem R⁵ und R⁶ eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden,
wobei bevorzugt R⁵, R⁶, R⁷, R⁷' unabhängig voneinander jeweils aus der Gruppe bestehend aus C₁-C₆-Alkyl, wobei C₁-C₆-Alkyl durch ein oder mehrere, nicht direkt zueinander benachbarte zweiwertige Reste ausgewählt aus der Gruppe bestehend aus Sauerstoff, Imino unterbrochen sein kann, ausgewählt sind, und wobei außerdem R⁵ und R⁶ eine C₁-C₁₀-Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden,
wobei bevorzugter R⁵, R⁶, R⁷, R⁷' unabhängig voneinander jeweils aus der Gruppe bestehend aus C₁-C₅-Alkyl, wobei C₁-C₅-Alkyl durch ein oder mehrere, nicht direkt zueinander benachbarte zweiwertige Reste ausgewählt aus der Gruppe bestehend aus Sauerstoff, Imino unterbrochen sein kann, ausgewählt sind, und wobei außerdem R⁵ und R⁶ eine C₁-C₆-Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden,
wobei noch mehr bevorzugter R⁵, R⁶, R⁷, R⁷' unabhängig voneinander jeweils aus der Gruppe bestehend aus Methyl, Ethyl, *n*-Propyl, *n*-Butyl, -CH₂CH₂(NH)CH₃, -CH₂CH₂(NCH₃)CH₂CH₂(NH)CH₃ ausgewählt sind, und wobei außerdem R⁵ und R⁶ zusammen eine Gruppe ausgewählt aus Piperidin, Morpholin, Piperazin, Pyrrolidin bilden,
wobei noch viel mehr bevorzugter R⁵, R⁶, R⁷, R⁷' unabhängig voneinander jeweils aus der Gruppe bestehend aus Methyl, Ethyl ausgewählt sind und am allerbevorzugtesten R⁵, R⁶, R⁷, R⁷' jeweils Methyl sind,
wobei R⁸ eine (m+1)-wertige organische Brückengruppe ist, die mit ein oder mehreren Resten ausgewählt aus der Gruppe bestehend aus Alkyl, Hydroxy, Nitro, Carboxyl, Carboxylester, Carboxylamid, Sulfonat, Sulfonsäureester, Sulfonamid, Halogen substituiert sein kann, und die durch ein oder mehrere, nicht direkt zueinander benachbarte zweiwertige Reste ausgewählt aus der Gruppe bestehend aus Sauerstoff, Imino unterbrochen sein kann,
wobei R⁸ insbesondere eine Alkylengruppe, die mit ein oder mehreren Resten ausgewählt aus der Gruppe bestehend aus Alkyl, Hydroxy, Nitro, Carboxyl, Carboxylester, Carboxylamid, Sulfonat, Sulfonsäureester, Sulfonamid, Halogen substituiert sein kann und die durch ein oder mehrere, nicht direkt zueinander benachbarte zweiwertige Reste ausgewählt aus der Gruppe bestehend aus Sauerstoff, Imino unterbrochen sein kann, ist,
wobei R⁸ bevorzugt eine Alkylengruppe, die durch ein oder mehrere, nicht direkt zueinander benachbarte zweiwertige Reste ausgewählt aus der Gruppe bestehend aus Sauerstoff, Imino unterbrochen sein kann, ist,
wobei R⁸ bevorzugter eine Alkylengruppe ist, noch bevorzugter eine C₁-C₁₀-Alkylengruppe, noch mehr bevorzugter eine C₁-C₆-Alkylengruppe, noch viel mehr bevorzugter eine C₂-C₆-Alkylengruppe, am bevorzugtesten aus der Gruppe bestehend aus Ethylen (also "-CH₂CH₂-), Butylen (also "-CH₂CH₂CH₂CH₂-"), Hexamethylen (also "-CH₂CH₂CH₂CH₂CH₂CH₂-") ausgewählt ist, und am allerbevorzugtesten Hexamethylen ist,
wobei m eine ganze Zahl von 1 bis 5, insbesondere 1 bis 4, noch bevorzugter 1 bis 3, noch mehr bevorzugter 1 bis 2 und am bevorzugtesten 1 ist,
gekennzeichnet dadurch, dass gasförmiges Methylchlorid in eine Schmelze **S** mindestens einer Verbindung der Formel **(II-A)** oder **(II-B),** bevorzugt Formel **(II-A),** mit 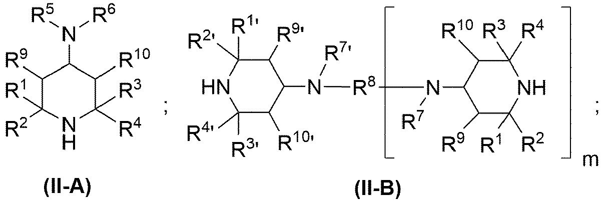
geleitet wird, so dass sich Methylchlorid und die mindestens eine Verbindung der Formel **(II-A)** oder **(II-B),** bevorzugt der Formel **(II-A),** zur Verbindung **(I-A)** bzw. **(I-B)** umsetzen.

Im erfindungsgemäßen Verfahren wird eine Verbindung der Formel **(II-A)** oder **(II-B)** mit Methylchlorid CH₃-Cl (CAS-Nummer: 74-87-3) zu einer Verbindung der Formel **(I-A)** bzw. **(I-B)** umgesetzt. Beim Einsatz einer Verbindung der Formel **(II-A)** wird eine Verbindung der Formel **(I-A)** erhalten, beim Einsatz einer Verbindung der Formel **(II-B)** wird eine Verbindung der Formel **(I-B)** erhalten.

In einer bevorzugten Ausführungsform wird im erfindungsgemäßen Verfahren mindestens eine Verbindung der Formel **(II-A)** eingesetzt, und so eine Verbindung der Formel **(I-A)** erhalten. In dieser bevorzugten Ausführungsform, in der im erfindungsgemäßen Verfahren mindestens eine Verbindung der Formel **(II-A)** eingesetzt wird, sind:
R¹, R², R³, R⁴ unabhängig voneinander jeweils ein C₁-C₆-Alkylrest, insbesondere ein C₁-C₄-Alkylrest, bevorzugt ein C₁-C₂-Alkylrest, und bevorzugter alle jeweils Methyl sind,
R⁹, R¹⁰ unabhängig voneinander jeweils aus der Gruppe bestehend aus Wasserstoff, C₁-C₆-Alkylrest, insbesondere aus der Gruppe bestehend aus Wasserstoff, C₁-C₄-Alkylrest, bevorzugt aus der Gruppe bestehend aus Wasserstoff, Methyl, Ethyl ausgewählt, und bevorzugter alle Wasserstoff sind,
R⁵, R⁶ unabhängig voneinander jeweils aus der Gruppe bestehend aus Wasserstoff, Alkyl, Cycloalkyl, Aryl, Aralkyl, Heterocyclyl ausgewählt, wobei die Reste Alkyl, Cycloalkyl, Aryl, Aralkyl, Heterocyclyl mit ein oder mehreren Resten ausgewählt aus der Gruppe bestehend aus Alkoxy, Hydroxy, Carboxyl, Carboxylester, Carboxylamid, Sulfonat, Sulfonsäureester, Sulfonamid, Halogen substituiert sein können, wobei außerdem Alkyl durch ein oder mehrere, nicht direkt zueinander benachbarte zweiwertige Reste ausgewählt aus der Gruppe bestehend aus Sauerstoff, Imino unterbrochen sein kann, und wobei außerdem R⁵ und R⁶ zusammen eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können und
wobei insbesondere R⁵, R⁶ unabhängig voneinander jeweils aus der Gruppe bestehend aus Wasserstoff, Alkyl, wobei Alkyl durch ein oder mehrere, nicht direkt zueinander benachbarte zweiwertige Reste ausgewählt aus der Gruppe bestehend aus Sauerstoff, Imino unterbrochen sein kann, ausgewählt sind, wobei außerdem R⁵ und R⁶ eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden,
wobei bevorzugt R⁵, R⁶ unabhängig voneinander jeweils aus der Gruppe bestehend aus Wasserstoff, C₁-C₆-Alkyl, wobei C₁-C₆-Alkyl durch ein oder mehrere, nicht direkt zueinander benachbarte zweiwertige Reste ausgewählt aus der Gruppe bestehend aus Sauerstoff, Imino unterbrochen sein kann, ausgewählt sind, und wobei außerdem R⁵ und R⁶ eine C₁-C₁₀-Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden,
wobei bevorzugter R⁵, R⁶ unabhängig voneinander jeweils aus der Gruppe bestehend aus Wasserstoff, C₁-C₄-Alkyl, wobei C₁-C₄-Alkyl durch ein oder mehrere, nicht direkt zueinander benachbarte zweiwertige Reste ausgewählt aus der Gruppe bestehend aus Sauerstoff, Imino unterbrochen sein kann, ausgewählt sind, und wobei außerdem R⁵ und R⁶ eine C₁-C₆-Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden,
wobei noch bevorzugter R⁵, R⁶ unabhängig voneinander jeweils eine C₁-C₅-Alkylgruppe, die durch ein oder mehrere, nicht direkt zueinander benachbarte zweiwertige Reste ausgewählt aus der Gruppe bestehend aus Sauerstoff, Imino unterbrochen sein kann, sind, und wobei außerdem R⁵ und R⁶ eine C₁-C₆-Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden,
wobei noch mehr bevorzugter R⁵, R⁶ unabhängig voneinander jeweils aus der Gruppe bestehend aus Methyl, Ethyl, *n*-Propyl, *n*-Butyl, -CH₂CH₂(NH)CH₃, -CH₂CH₂(NCH₃)CH₂CH₂(NH)CH₃ ausgewählt sind, und wobei außerdem R⁵ und R⁶ zusammen eine Gruppe ausgewählt aus Piperidin, Morpholin, Piperazin, Pyrrolidin bilden,
wobei noch viel mehr bevorzugter R⁵, R⁶ unabhängig voneinander jeweils aus der Gruppe bestehend aus Methyl, Ethyl ausgewählt sind und am allerbevorzugtesten R⁵, R⁶ jeweils Methyl sind.

"Schmelze" bedeutet dabei erfindungsgemäß, dass das eingesetzte Edukt, also die mindestens eine im erfindungsgemäßen Verfahren eingesetzte Verbindung der Formeln **(II-A)** oder **(II-B),** nicht vollständig in einem Lösungsmittel gelöst eingesetzt wird, sondern mindestens teilweise ungelöst oberhalb ihres Schmelzpunktes, in flüssigem Aggregatzustand vorliegt.

Dies umschließt auch die Fälle, in denen im erfindungsgemäßen Verfahren die Schmelze **S** mindestens einer Verbindung der Formeln **(II-A)** und **(II-B)** als Mischung **M** mit mindestens einem aprotischen organischen Lösungsmittel eingesetzt wird, solange nur alle eingesetzten aprotischen organischen Lösungsmittel in einer solchen Menge vorliegen, dass sich die Schmelze **S** nicht vollständig in ihnen löst.

Insbesondere bedeutet dies demnach, dass die Menge aller von der Mischung **M** umfassten aprotischen organischen Lösungsmittel so eingestellt wird, dass mindestens 50 Mol.-% aller von der Mischung **M** umfassten Verbindungen der Formeln **(II-A)** und **(II-B)** als Schmelze S (also ungelöst, aber in flüssigem Aggregatzustand) vorliegen. Bevorzugt bedeutet dies, dass die Menge aller von der Mischung **M** umfassten aprotischen organischen Lösungsmittel so eingestellt wird, dass mindestens 60 Mol.-%, bevorzugter mindestens 70 Mol.-%, noch bevorzugter mindestens 80 Mol.-%, noch mehr bevorzugter mindestens 90 Mol.-%, noch viel mehr bevorzugter mindestens 95 Mol.-%, noch viel mehr bevorzugter mindestens 99 Mol.-% aller von der Mischung **M** umfassten Verbindungen der Formeln **(II-A)** und **(II-B)** als Schmelze **S** vorliegen.

In der bevorzugten Ausführungsform, in der im erfindungsgemäßen Verfahren mindestens eine Verbindung der Formel **(II-A)** eingesetzt wird, bedeutet dies insbesondere, dass die Menge aller von der Mischung **M** umfassten aprotischen organischen Lösungsmittel so eingestellt wird, dass mindestens 50 Mol.-% aller von der Mischung **M** umfassten Verbindungen der Formeln **(II-A)** als Schmelze **S** (also ungelöst, aber in flüssigem Aggregatzustand) vorliegen. Bevorzugt bedeutet dies, dass dann die Menge aller von der Mischung **M** umfassten aprotischen organischen Lösungsmittel so eingestellt wird, dass mindestens 60 Mol.-%, bevorzugter mindestens 70 Mol.-%, noch bevorzugter mindestens 80 Mol.-%, noch mehr bevorzugter mindestens 90 Mol.-%, noch viel mehr bevorzugter mindestens 95 Mol.-%, noch viel mehr bevorzugter mindestens 99 Mol.-% aller von der Mischung **M** umfassten Verbindungen der Formeln **(II-A)** als Schmelze **S** vorliegen.

Insbesondere wird das erfindungsgemäße Verfahren dabei bei einer Temperatur von 0 °C bis 200 °C, bevorzugt 15 °C bis 150 °C, bevorzugter 25 bis 100 °C, noch bevorzugter 40 °C bis 100 °C, noch mehr bevorzugter 40 bis 75 °C, am bevorzugtesten 60 °C bis 75 °C durchgeführt.

Insbesondere wird das erfindungsgemäße Verfahren dabei bei einem Druck von 0.5 bar bis 10 bar, bevorzugt bei 1 bar bis 6 bar, bevorzugter bei 1 bar bis 2 bar, am bevorzugtesten bei 1 bar (= Atmosphärendruck) durchgeführt. Die Umsetzung kann in Anwesenheit oder Abwesenheit einer Base durchgeführt werden, bevorzugt aber in Abwesenheit einer Base. Beispiele für geeignete Basen sind anionische lonenaustauscher, Alkalihydroxide, wie Natriumhydroxid oder Kaliumhydroxid, oder organische Amine, vorzugsweise solche Amine, die bei den Drucken und Temperaturen in der Umsetzung als flüssige Phase vorliegen.

Im erfindungsgemäßen Verfahren wird überraschend eine effiziente Umsetzung der Edukte beobachtet, was sich darin äußert, dass mit der gleichen molaren Menge an Methylchlorid mehr Produkt der Formel **(I-A)** bzw. **(I-B)** pro Zeiteinheit erhalten wird als in den Verfahren des Standes der Technik.

Bei der Methylierung der Verbindungen der Struktur **(II-A)** und **(II-B)** mit Hilfe von Methylchlorid besteht das Problem, dass dieses im bevorzugten Temperaturbereich bei Normaldruck gasförmig ist. Beim Vorgehen gemäß den Verfahren des Standes der Technik, in denen die entsprechenden Verbindungen der Struktur **(II-A)** bzw. **(II-B)** (Beispiel 4, Seite 31 der WO 2008/028830 A1) methyliert werden sollen, ist es deshalb nötig, das entsprechende Alkylierungsmittel lange durch die Reaktionsmischung zu leiten, um eine befriedigende Ausbeute an methyliertem Produkt zu erhalten. Um unnötige Verluste zu vermeiden, ist eine langsame Dosierung nötig. Gerade im Falle von Methylchlorid ist dies ein großes Problem, da dessen Siedepunkt mit - 24.2 °C unterhalb des für die Laborsynthese bevorzugten Bereichs liegt und es somit in diesem Bereich gasförmig ist. Bei zu großer Dosierung gast Methylchlorid aus der Reaktionsmischung aus, ohne vollständig mit der Verbindung der Struktur **(II-A)** bzw. **(II-B)** zu reagieren. Dieses Problem kann man zwar durch Durchführung der Reaktion in einem Autoklaven abmildern, aber dies bedeutet einen hohen Aufwand.

Das erfindungsgemäße Verfahren erlaubt einen sparsameren Einsatz des Alkylierungsmittels Methylchlorid als im Stand der Technik, in denen die Verbindung **(II-A)** und **(II-B)** vollständig im Lösungsmittel gelöst eingesetzt wird. Ein Verfahren, in denen das Edukt *N*,*N*,2,2,6,6-Hexamethylpiperidin-4-amin im Alkylierungsmittel gelöst eingesetzt wird, ist in Beispiel 3, Seite 30 der WO 2008/028830 A1 beschrieben. Hier erfolgt allerdings eine Umsetzung mit flüssigem 1-Chlorpropan, bei dem das beschriebene Problem gar nicht erst auftritt, da es im bevorzugten Bereich flüssig ist.

Das erfindungsgemäße Verfahren beruht hingegen darauf, dass man das Alkylierungsmittel Methylchlorid gasförmig durch die Schmelze **S** leitet. Dadurch wird der mögliche Kontakt zwischen dem entsprechenden Alkylhalogenid und der Verbindung der Struktur **(II-A)** und **(II-B)** optimiert, was zu einer schnelleren und vollständigeren Reaktion zum Produkt der Verbindung (I-A) bzw. **(I-B)** führt. Das Alkylierungsmittel wird optimal eingesetzt und die Verluste minimiert.

Da das Produkt ein Salz ist, fällt es aus der Reaktionsmischung aus und ist so leicht abzutrennen. Die Entfernung von Lösungsmitteln oder überschüssigem Edukt (etwa wenn dieses auch als Lösungsmittel fungiert) entfällt. Eventuell bei der Entfernung des Niederschlages mitgeschlepptes Edukt der Struktur **(II-A)** und **(II-B)** kann direkt wieder der Reaktionsmischung zugeführt werden.

Im erfindungsgemäßen Verfahren liegt insbesondere das molare Verhältnis des Gesamtgewichts des eingesetzten Methylchlorids zum Gesamtgewicht aller eingesetzten Verbindungen der Formel **(II-A)** und **(II-B)** im Bereich 20 : 1 bis 1 : 20, bevorzugt 9 : 1 bis 1 : 4, bevorzugter 5 : 1 bis 1 : 2, noch bevorzugter 3 : 1 bis 1 : 1, am bevorzugtesten 1.05 : 1 bis 1 : 1.

In einer alternativen bevorzugten Ausführungsform wird die Menge des im erfindungsgemäßen Verfahren eingesetzten Methylchlorids bezogen auf die Menge der im erfindungsgemäßen Verfahrens eingesetzten Verbindungen der Formel **(II-A)** bzw. **(II-B)** bestimmt.

In dieser alternativen bevorzugten Ausführungsform werden pro Mol an im erfindungsgemäßen Verfahren eingesetzter Verbindung der Formel **(II-A)** 0.05 bis 20 Mol Methylchlorid, bevorzugt 0.25 bis 9 Mol Methylchlorid, bevorzugter 0.5 bis 5 Mol Methylchlorid, noch bevorzugter 1 bis 3 Mol Methylchlorid, am bevorzugtesten 1.0 bis 1.05 Mol Methylchlorid eingesetzt und pro Mol an im erfindungsgemäßen Verfahren eingesetzter Verbindung der Formel **(II-B)** (m+1) x [0.05 bis 20] Mol Methylchlorid, bevorzugt (m+1) x [0.25 bis 9] Mol Methylchlorid, bevorzugter (m+1) x [0.5 bis 5] Mol Methylchlorid, noch bevorzugter (m+1) x [1 bis 3] Mol Methylchlorid, am bevorzugtesten (m+1) x [1.0 bis 1.05] Mol Methylchlorid eingesetzt.

Es ist selbstverständlich dass, wenn im erfindungsgemäßen Verfahren mehrere Verbindungen der Formel **(II-B),** welche sich durch den Wert von m unterscheiden, eingesetzt werden, dass dann die bevorzugte Menge an Methylchlorid für jede Gruppe mit dem selben Wert für m wiederholt werden und dann die pro Gruppe berechneten Mengen an Methylchlorid addiert werden müssen.

In der bevorzugten Ausführungsform, in der im erfindungsgemäßen Verfahren mindestens eine Verbindung der Formel **(II-A)** eingesetzt wird, bedeutet dies insbesondere, dass das molare Verhältnis des Gesamtgewichts des eingesetzten Methylchlorids zum Gesamtgewicht aller eingesetzten Verbindungen der Formel **(II-A)** im Bereich 20 : 1 bis 1 : 20, bevorzugt 9 : 1 bis 1 : 4, bevorzugter 5 : 1 bis 1 : 2, noch bevorzugter 3 : 1 bis 1 : 1, am bevorzugtesten 1.05 : 1 bis 1 : 1 liegt.

Nach Beendigung des erfindungsgemäßen Verfahrens können die erhaltenen Verbindungen der Struktur **(I-A)** und **(I-B),** bevorzugt **(I-A),** nach dem Fachmann bekannten Verfahren aufgearbeitet werden.

In einer bevorzugten Ausführungsform des erfindungsgemäßen Verfahrens fällt die erhaltene mindestens eine Verbindung der Formel **(I-A)** bzw. **(I-B)** mindestens teilweise als Niederschlag aus der Schmelze **S** aus und wird mindestens teilweise als Fraktion **F** aus der Schmelze **S** abgetrennt. Insbesondere wird bei dieser Abtrennung auch Edukt, also mindestens eine Verbindung der Formel **(II-A)** bzw. **(II-B),** aus der Schmelze **S** mitgeschleppt, so dass die Fraktion auch mindestens eine Verbindung der Formel **(II-A)** bzw. **(II-B)** umfasst. In dieser besonderen Ausführungsform wird die Fraktion **F** mindestens anteilig in mindestens ein Produkt der Formel **(I-A)** oder **(I-B)** und mindestens ein Edukt der Formel **(II-A)** oder **(II-B)** aufgetrennt und die aus dieser Fraktion abgetrennte mindestens eine Verbindung der Formel **(II-A)** bzw. **(II-B)** wieder in die Schmelze **S** rückgeführt.

Das erfindungsgemäße Verfahren eignet sich dazu, kontinuierlich durchgeführt zu werden. Es ist deshalb bevorzugt, das erfindungsgemäße Verfahren kontinuierlich durchzuführen. Dies kann etwa in einem Schlaufenreaktor durchgeführt werden, in dem kontinuierlich durch einen Strom der Schmelze **S** Methylchlorid geleitet wird und das ausfallende mindestens eine Produkt der Formel **(I-A)** und **(I-B)** ausgefällt, als Fraktion F abgetrennt, und eventuell mit abgetrenntem mindestens einem Edukt der Formel **(II-A)** bzw. **(II-B)** wieder in die Schmelze **S** rückgeführt wird.

Die mit dem erfindungsgemäßen Verfahren erhaltenen Verbindungen der Formel **(I)** werden im Anschluss an das erfindungsgemäße Verfahren typischerweise am endocyclischen Stickstoffatom zum Nitroxylradikal N-O· oxidiert. Diese Reaktion ist dem Fachmann geläufig sind und beispielsweise in der WO 2018/028830 A1 beschrieben.

### Allgemeine Begriffe

R⁸ ist eine (m+1)-wertige organische Brückengruppe, wobei m eine ganze Zahl zwischen 1 und 5 ist. Darunter ist demnach ein organischer Rest zu verstehen, der über zwei, drei, vier, fünf oder sechs kovalente Bindungen mit dem Rest des Moleküls verbunden ist. R⁸ ist bevorzugt Alkylen.

Beispiele für zweiwertige organische Brückengruppen sind Alkylen, Alkylenoxy, Poly(alkylenoxy), Alkylenamino, Poly(alkylenamino), Cycloalkylen, Arylen, Aralkylen oder Heterocyclylen.

Alkylengruppen können sowohl verzweigt als auch unverzweigt sein. Eine Alkylengruppe enthält typischerweise ein bis zu zwanzig Kohlenstoffatome, bevorzugt zwei bis zu sechs Kohlenstoffatome. Beispiele für Alkylengruppen sind: Methylen, Ethylen, Propylen und Butylen, Pentylen, Hexylen. Alkylengruppen können gegebenenfalls substituiert sein, beispielsweise mit Alkyl, Alkoxy, Hydroxy, Carboxyl, Carboxylester, Carboxylamid, Sulfonat, Sulfonsäureester, Sulfonamid, Halogen.

Alkylenoxy- und Poly(alkylenoxy)gruppen können sowohl verzweigte als auch unverzweigte Alkylengruppen enthalten. Eine in einer Alkylenoxy- oder in einer Poly(alkylenoxy)gruppe auftretende Alkylengruppe enthält typischerweise zwei bis vier Kohlenstoffatome, bevorzugt zwei oder drei Kohlenstoffatome. Die Anzahl der Wiederholeinheiten in den Poly(alkylenoxy)gruppen kann in weiten Bereichen schwanken. Typische Anzahlen von Wiederholeinheiten bewegen sich im Bereich von 2 bis 50. Beispiele für Alkylenoxygruppen sind: Ethylenoxy, Propylenoxy und Butylenoxy. Beispiele für Poly(alkylenoxy)gruppen sind: Poly(ethylenoxy), Poly(propylenoxy) und Poly(butylenoxy).

Alkylenamino- und Poly(alkylenamino)gruppen können sowohl verzweigte als auch unverzweigte Alkylengruppen enthalten. Eine in einer Alkylenamino- oder in einer Poly(alkylenamino)gruppe auftretende Alkylengruppe enthält typischerweise zwei bis vier Kohlenstoffatome, bevorzugt zwei oder drei Kohlenstoffatome. Die Anzahl der Wiederholeinheiten in den Poly(alkylenamino)gruppen kann in weiten Bereichen schwanken. Typische Anzahlen von Wiederholeinheiten bewegen sich im Bereich von 2 bis 50. Beispiele für Alkylenaminogruppen sind: Ethylenamino, Propylenamino und Butylenamino. Beispiele für Poly(alkylenamino)gruppen sind: Poly(ethylenamino), Poly(propylenamino) und Poly(butylenamino).

Cycloalkylengruppen enthalten typischerweise fünf, sechs oder sieben Ringkohlenstoffatome, die jeweils unabhängig voneinander substituiert sein können. Beispiele für Substituenten sind Alkylgruppen oder zwei Alkylgruppen, die zusammen mit den Ringkohlenstoffen, an denen sie gebunden sind, einen weiteren Ring bilden können. Ein Beispiel für eine Cycloalkylengruppe ist Cyclohexylen.

Arylengruppen sind typischerweise cyclische aromatische Gruppen, die fünf bis vierzehn Kohlenstoffatome enthalten, die jeweils unabhängig voneinander substituiert sein können. Beispiele für Arylengruppen sind o-Phenylen, m-Phenylen, p-Phenyl, Naphthylengruppen oder Biphenylengruppen.

Heterocyclylgruppen sind typischerweise cyclische Gruppen mit vier bis zehn Ringkohlenstoffatomen und mindestens einem Ringheteroatom, die jeweils unabhängig voneinander substituiert sein können, insbesondere mit Alkyl. Beispiele für Heteroatome sind Sauerstoff, Stickstoff, Phosphor, Bor, Selen oder Schwefel. Beispiele für Heterocyclylengruppen sind Furandiyl, Thiophendiyl, Pyrroldiyl oder Imidazoldiyl. Heterocyclylengruppen sind vorzugsweise aromatisch.

Aralkylengruppen sind typischerweise Arylgruppen, an die ein oder zwei Alkylgruppen kovalent gebunden sind. Aralkylgruppen können über ihren Arylrest und ihren Alkylrest oder über zwei Alkylreste mit dem Rest des Moleküls kovalent verbunden sein. Die Aralkylengruppe kann am aromatischen Ring beispielsweise mit Alkylgruppen oder mit Halogenatomen substituiert sein. Beispiele für Aralkylengruppen sind Benzylen oder Dimethylphenylen (Xylylen).

Beispiele für dreiwertige organische Reste R⁸ sind Alkyltriyl, Alkoxytriyl, Trispoly(alkylenoxy), Tris-poly(alkylenamino), Cycloalkyltriyl, Aryltriyl, Aralkyltriyl oder Heterocyclyltriyl. Diese Reste entsprechen den bereits weiter oben eingehend beschriebenen zweiwertigen Resten mit dem Unterschied, dass diese mit drei kovalenten Bindungen anstelle von zwei kovalenten Bindungen mit dem Rest des Moleküls verbunden sind.

Beispiele für vierwertige organische Reste R⁸ sind Alkylquaternyl, Alkoxyquaternyl, Quater-poly(alkylenoxy), Quaterpoly(alkylenamino), Cycloalkylquaternyl, Arylquaternyl, Aralkylquaternyl oder Heterocyclylquaternyl. Diese Reste entsprechen den bereits weiter oben eingehend beschriebenen zweiwertigen Resten mit dem Unterschied, dass diese mit vier kovalenten Bindungen anstelle von zwei kovalenten Bindungen mit dem Rest des Moleküls verbunden sind.

Beispiele für fünfwertige organische Reste R⁸ sind Alkylquinquinyl, Alkoxyquinquinyl, Quinqui-poly(alkylenoxy), Quinquipoly(alkylenamino), Cycloalkylquinquinyl, Arylquinquinyl, Aralkylquinquinyl oder Heterocyclylquinquinyl. Diese Reste entsprechen den bereits weiter oben eingehend beschriebenen zweiwertigen Resten mit dem Unterschied, dass diese mit fünf kovalenten Bindungen anstelle von zwei kovalenten Bindungen mit dem Rest des Moleküls verbunden sind.

Beispiele für sechswertige organische Reste R⁸ sind Alkylhexyl, Alkoxyhexyl, Hexyl-poly(alkylenoxy), Hexylpoly(alkylenamino), Cycloalkylhexyl, Arylhexyl, Aralkylhexyl oder Heterocyclylhexyl. Diese Reste entsprechen den bereits weiter oben eingehend beschriebenen zweiwertigen Resten mit dem Unterschied, dass diese mit sechs kovalenten Bindungen anstelle von zwei kovalenten Bindungen mit dem Rest des Moleküls verbunden sind.

In einer bevorzugten Ausführungsform der vorliegenden Erfindung sind R⁵, R⁶, R⁷ und R⁷' unabhängig voneinander jeweils Alkyl, welches gegebenenfalls mit ein oder mehreren Resten ausgewählt aus der Gruppe bestehend aus Alkoxy, Hydroxy, Carboxyl, Carboxylester, Carboxylamid, Sulfonat, Sulfonsäureester, Sulfonamid oder Halogen substituiert sind und/oder die gegebenenfalls in der Alkylgruppe durch ein oder mehrere, nicht direkt zueinander benachbarte zweiwertige Reste ausgewählt aus der Gruppe bestehend aus Sauerstoff, Imino unterbrochen sind.

Ganz besonders bevorzugt bedeuten R⁵, R⁶, R⁷ und R⁷' unabhängig voneinander C₁-C₆- Alkyl.

Bedeutet einer der Reste R⁵, R⁶, R⁷ und/oder R⁷' Alkyl, so kann die Alkylgruppe sowohl verzweigt als auch unverzweigt sein. Eine Alkylgruppe dieser Reste enthält typischerweise ein bis zu zwanzig Kohlenstoffatome, bevorzugt ein bis zu zehn Kohlenstoffatome. Beispiele für Alkylgruppen sind: Methyl, Ethyl, n-Propyl, iso-Propyl, n-Butyl, sec-Butyl, tert-Butyl, Pentyl, n-Hexyl, n-Heptyl, 2-Ethylhexyl, n-Octyl, n-Nonyl, n-Decyl, n-Undecyl, n-Dodecyl, n-Tridecyl, n-Tetradecyl, n-Pentadecyl, n- Hexadecyl, n-Heptadecyl, n-Octadecyl, n-Nonadecyl oder Eicosyl. Besonders bevorzugt sind von den vorgenannten Alkylgruppen jene mit ein bis sechs, noch bevorzugter jene mit ein bis vier Kohlenstoffatomen.

Die Reste R⁵, R⁶, R⁷ und/oder R⁷' als Alkyl können gegebenenfalls substituiert sein, beispielsweise mit Alkoxy, Hydroxy, Carboxyl, Carboxylester, Carboxylamid, Sulfonat, Sulfonsäureester, Sulfonamid oder Halogen, bevorzugt mit Alkoxy, Hydroxy, Halogen. Der Rest R⁷ als Alkyl kann gegebenenfalls auch mit Nitro substituiert sein.

Die Reste R⁵, R⁶, R⁷ und/oder R⁷' als Alkyl können gegebenenfalls durch ein oder mehrere, nicht direkt zueinander benachbarte zweiwertige Sauerstoff- oder Iminoreste unterbrochen sein.

Beispiele dafür sind einwertige Reste abgeleitet von Polyethylenglykolen, von Polypropylenglykolen, von Polyethyleniminen oder von Polypropyleniminen.

Bedeutet einer der Reste R⁵, R⁶, R⁷ und/oder R⁷' Cycloalkyl, so ist die Cycloalkylgruppe typischerweise eine cyclische Gruppe, enthaltend fünf bis acht, vorzugsweise fünf, sechs oder sieben Ringkohlenstoffatome, die jeweils unabhängig voneinander substituiert sein können. Beispiele für Substituenten sind Alkylgruppen oder zwei Alkylgruppen, die zusammen mit den Ringkohlenstoffen, an denen sie gebunden sind, einen weiteren Ring bilden können. Beispiele für Cycloalkylgruppen sind Cyclopentyl oder Cyclohexyl. Cycloalkylgruppen können gegebenenfalls mit Alkyl, Alkoxy, Hydroxy, Carboxyl, Carboxylester, Carboxylamid, Sulfonat, Sulfonsäureester, Sulfonamid oder Halogen substituiert sein.

Bedeutet einer der Reste R⁵, R⁶, R⁷ und/oder R⁷' Aryl, so ist die Arylgruppe typischerweise eine cyclische aromatische Gruppe, enthaltend fünf bis vierzehn Kohlenstoffatome, die jeweils unabhängig voneinander substituiert sein können. Beispiele für Substituenten sind Alkylgruppen oder zwei Alkylgruppen, die gemeinsam mit den Ringkohlenstoffatomen, an denen sie gebunden sind, einen weiteren Ring bilden können. Beispiele für Arylgruppen sind Phenyl, Biphenyl, Anthryl oder Phenantolyl. Arylgruppen können gegebenenfalls mit Alkyl, Alkoxy, Hydroxy, Carboxyl, Carboxylester, Carboxylamid, Sulfonat, Sulfonsäureester, Sulfonamid oder Halogen substituiert sein.

Bedeutet einer der Reste R⁵, R⁶, R⁷ und/oder R⁷' Heterocyclyl, so kann die Heterocyclylgruppe typischerweise eine cyclische Gruppe mit vier bis zehn Ringkohlenstoffatomen und mindestens einem Ringheteroatom aufweisen, die jeweils unabhängig voneinander substituiert sein können.

Beispiele für Substituenten der Heterocyclylgruppe sind Alkylgruppen oder zwei Alkylgruppen die zusammen mit den Ringkohlenstoffen an denen sie gebunden sind einen weiteren Ring bilden können. Beispiele für Heteroatome sind Sauerstoff, Stickstoff, Phosphor, Bor, Selen oder Schwefel. Beispiele für Heterocyclyigruppen sind Furyl, Thienyl, Pyrrolyl oder Imidazolyl. Heterocyclyigruppen sind vorzugsweise aromatisch. Heterocyclyigruppen können gegebenenfalls mit Alkyl, Alkoxy, Hydroxy, Carboxyl, Carboxylester, Carboxylamid, Sulfonat, Sulfonsäureester, Sulfonamid oder Halogen substituiert sein.

Bedeutet einer der Reste R⁵, R⁶, R⁷ und/oder R⁷' Aralkyl, so ist die Aralkylgruppe typischerweise eine Arylgruppe, wobei Aryl zuvor bereits definiert wurde, an die kovalent eine Alkylgruppe gebunden ist. Die Aralkylgruppe kann am aromatischen Ring beispielsweise mit Alkylgruppen oder mit Halogenatomen substituiert sein. Ein Beispiel für eine Aralkylgruppe ist Benzyl. Aralkylgruppen können gegebenenfalls mit Alkoxy, Hydroxy, Carboxyl, Carboxylester, Carboxylamid, Sulfonat, Sulfonsäureester, Sulfonamid oder Halogen, bevorzugt mit Alkoxy, Hydroxy, Halogen substituiert sein.

Aprotische organische Lösungsmittel sind insbesondere aus der Gruppe bestehend aus Ether, Nitril, halogenierter Kohlenwasserstoff, aromatischer Kohlenwasserstoff, aliphatischer Kohlenwasserstoff ausgewählt.

Bevorzugte Ether sind Diethylether und Di-isopropylether. Beispiele für Nitrile sind Acetonitril. Bevorzugte halogenierte Kohlenwasserstoffe sind chlorierte und/oder fluorierte aliphatische Kohlenwasserstoffe, wie Dichlormethan, Chloroform, Tetrachlorkohlenstoff, Dichlorethan oder Chlorfluorethan.

Bevorzugte aromatische Kohlenwasserstoffe sind Benzol, Toluol oder Xylol. Beispiele für aliphatische Kohlenwasserstoffe sind Hexan, Octan oder Decan sowie Petrolether oder Petroleum.

### Beispiele

### Erfinderisches Beispiel E1 und Vergleichsbeispiel V1

100 g (0.5 mol) *N,N*,2,2,6,6-Hexamethylpiperidin-4-amin werden bei Normaldruck in einem Kolben mit Diaphragma und angeschlossenem Blasenzähler auf 60 °C erhitzt und liegen bei dieser Temperatur im flüssigen Zustand vor. Im Falle des Vergleichsbeispiels **V1** werden 100 ml einer 1 :1 (*v*/*v*) - Mischung Acetonitril/ Toluol zugegeben. Im Falle des erfinderischen Beispiels **E1** wird kein Lösungsmittel zugegeben.

Unter Rühren werden dann in den Beispielen **E1, V1** jeweils ~ 7 I Chlormethan (~ 0.25 mol) als Gas durch die Lösung geleitet.

Nach Abkühlen auf Raumtemperatur und wird der Niederschlag abzentrifugiert und mit Toluol gewaschen. Die Menge des jeweils erhaltenen Produktes *N*,*N*,*N*,2,2,6,6-Heptamethylpiperidin-4-ammoniumchlorid ist im erfinderischen Beispiel **E1** deutlich höher als im Vergleichsbeispiele **V1.**

Dies zeigt, dass mit dem erfindungsgemäßen Verfahren ein deutlich effizienterer Einsatz an Chlormethan möglich ist, da mehr des gewünschten Produktes *N*,*N*,*N*,2,2,6,6-Heptamethylpiperidin-4-ammoniumchlorid erhalten wird.

## Patentansprüche

1. Verfahren zur Herstellung mindestens einer Verbindung der Formel **(I-A)** oder **(I-B)** mit 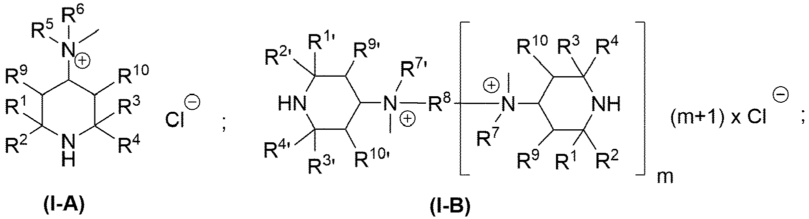
wobei R¹, R², R³, R⁴, R¹', R²', R³', R⁴' unabhängig voneinander jeweils ein C₁-C₆-Alkylrest sind,
wobei R⁹, R¹⁰, R⁹', R¹⁰' unabhängig voneinander jeweils aus der Gruppe bestehend aus Wasserstoff, C₁-C₆-Alkylrest ausgewählt sind,
wobei R⁵, R⁶, R⁷, R⁷' unabhängig voneinander jeweils aus der Gruppe bestehend aus Wasserstoff, Alkyl, Cycloalkyl, Aryl, Aralkyl, Heterocyclyl ausgewählt sind, wobei die Reste Alkyl, Cycloalkyl, Aryl, Aralkyl, Heterocyclyl mit ein oder mehreren Resten ausgewählt aus der Gruppe bestehend aus Alkoxy, Hydroxy, Carboxyl, Carboxylester, Carboxylamid, Sulfonat, Sulfonsäureester, Sulfonamid, Halogen substituiert sein können, wobei außerdem Alkyl durch ein oder mehrere, nicht direkt zueinander benachbarte zweiwertige Reste ausgewählt aus der Gruppe bestehend aus Sauerstoff, Imino unterbrochen sein kann, und wobei außerdem R⁵ und R⁶ zusammen eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können,
wobei R⁸ eine (m+1)-wertige organische Brückengruppe ist, die mit ein oder mehreren Resten ausgewählt aus der Gruppe bestehend aus Alkyl, Hydroxy, Nitro, Carboxyl, Carboxylester, Carboxylamid, Sulfonat, Sulfonsäureester, Sulfonamid, Halogen substituiert sein kann, und die durch ein oder mehrere, nicht direkt zueinander benachbarte zweiwertige Reste ausgewählt aus der Gruppe bestehend aus Sauerstoff, Imino unterbrochen sein kann,
wobei m eine ganze Zahl von 1 bis 5 ist,
**gekennzeichnet dadurch, dass** gasförmiges Methylchlorid in eine Schmelze **S** mindestens einer Verbindung der Formel **(II-A)** oder **(II-B)** mit 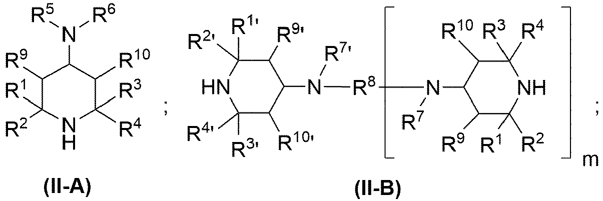
geleitet wird, so dass sich Methylchlorid und die mindestens eine Verbindung der Formel **(II-A)** oder **(II-B)** zur Verbindung **(I-A)** bzw. **(I-B)** umsetzen.

2. Verfahren nach Anspruch 1, wobei R¹, R², R³, R⁴, R¹', R²', R³', R⁴' unabhängig voneinander jeweils ein C₁-C₄-Alkylrest sind,
wobei R⁹, R¹⁰, R⁹', R¹⁰' unabhängig voneinander jeweils aus der Gruppe bestehend aus Wasserstoff, C₁-C₄-Alkylrest ausgewählt sind,
wobei R⁵, R⁶, R⁷, R⁷' unabhängig voneinander jeweils aus der Gruppe bestehend aus Wasserstoff, Alkyl, wobei Alkyl durch ein oder mehrere, nicht direkt zueinander benachbarte zweiwertige Reste ausgewählt aus der Gruppe bestehend aus Sauerstoff, Imino unterbrochen sein kann, ausgewählt sind, und wobei außerdem R⁵ und R⁶ eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können,
wobei R⁸ eine Alkylengruppe, die mit ein oder mehreren Resten ausgewählt aus der Gruppe bestehend aus Alkyl, Hydroxy, Nitro, Carboxyl, Carboxylester, Carboxylamid, Sulfonat, Sulfonsäureester, Sulfonamid, Halogen substituiert sein kann und die durch ein oder mehrere, nicht direkt zueinander benachbarte zweiwertige Reste ausgewählt aus der Gruppe bestehend aus Sauerstoff, Imino unterbrochen sein kann, ist,
wobei m = 1 ist.

3. Verfahren nach Anspruch 2, wobei R¹, R², R³, R⁴, R¹', R²', R³', R⁴', R⁵, R⁶, R⁷ jeweils Methyl sind, R⁹, R¹⁰, R⁹', R¹⁰' jeweils Wasserstoff sind, R⁸ aus der Gruppe bestehend aus Ethylen, Butylen, Hexamethylen ausgewählt ist, und m = 1 ist.

4. Verfahren nach einem der Ansprüche 1 bis 3, wobei die Schmelze **S** mindestens einer Verbindung der Formeln **(II-A)** und **(II-B)** als Mischung **M** mit mindestens einem aprotischen organischen Lösungsmittel eingesetzt wird, wobei die Gesamtmenge aller eingesetzten aprotischen organischen Lösungsmittel nicht ausreicht, um die Schmelze **S** darin zu lösen.

5. Verfahren nach Anspruch 4, wobei die Menge aller von der Mischung **M** umfassten aprotischen organischen Lösungsmittel so eingestellt wird, dass mindestens 50 Mol.-% aller von der Mischung **M** umfassten Verbindungen der Formeln **(II-A)** und **(II-B)** als Schmelze S vorliegen.

6. Verfahren nach einem der Ansprüche 1 bis 5, wobei die erhaltene mindestens eine Verbindung der Formel **(I-A)** bzw. **(I-B)** mindestens teilweise als Niederschlag aus der Schmelze **S** ausfällt und mindestens teilweise als Fraktion **F** aus der Schmelze **S** abgetrennt wird.

7. Verfahren nach Anspruch 6, wobei die Fraktion **F** auch mindestens eine Verbindung der Formel **(II-A)** bzw. **(II-B)** umfasst, mindestens anteilig in Produkt der Formel **(I-A)** oder **(I-B)** und mindestens ein Edukt der Formel **(II-A)** oder **(II-B)** aufgetrennt wird und die aus dieser Fraktion abgetrennte mindestens eine Verbindung der Formel **(II-A)** bzw. **(II-B)** wieder in die Schmelze S rückgeführt wird.

8. Verfahren nach einem der Ansprüche 1 bis 7, welches bei einer Temperatur von 0 °C bis 200 °C und einem Druck von 0.5 bar bis 10 bar durchgeführt wird.

9. Verfahren nach einem der Ansprüche 1 bis 8, welches kontinuierlich durchgeführt wird.

## Claims

1. Process for preparing at least one compound of the formula **(I-A)** or **(I-B)** 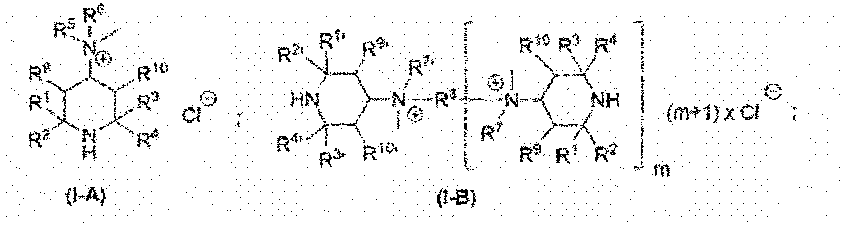
wherein R¹, R², R³, R⁴, R¹', R²', R³', R⁴' are each independently a C₁-C₆-alkyl radical,
wherein R⁹, R¹⁰, R⁹', R¹⁰' are each independently selected from the group consisting of hydrogen, C₁-C₆-alkyl radical,
wherein R⁵, R⁶, R⁷, R⁷' are each independently selected from the group consisting of hydrogen, alkyl, cycloalkyl, aryl, aralkyl, heterocyclyl, wherein the radicals alkyl, cycloalkyl, aryl, aralkyl, heterocyclyl may be substituted by one or more radicals selected from the group consisting of alkoxy, hydroxy, carboxyl, carboxyl ester, carboxyl amide, sulfonate, sulfonic acid ester, sulfonamide, halogen, where, in addition, alkyl may be interrupted by one or more divalent radicals not directly adjacent to one another selected from the group consisting of oxygen, imino, and where, in addition, R⁵ and R⁶ may together form an alkylene group which may be interrupted by one or more divalent radicals not directly adjacent to one another selected from the group consisting of oxygen, imino,
wherein R⁸ is an (m+1)-valent organic bridging group, which may be substituted by one or more radicals selected from the group consisting of alkyl, hydroxy, nitro, carboxyl, carboxyl ester, carboxyl amide, sulfonate, sulfonic acid ester, sulfonamide, halogen, and which may be interrupted by one or more divalent radicals not directly adjacent to one another selected from the group consisting of oxygen, imino,
where m is an integer from 1 to 5,
**characterized in that** gaseous methyl chloride is introduced into a melt **S** of at least one compound of the formula **(II-A)** or **(II-B)** 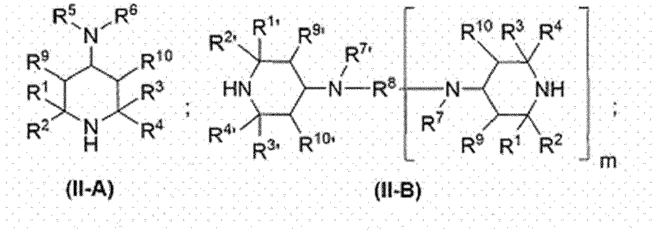
such that methyl chloride and the at least one compound of the formula **(II-A)** or **(II-B)** react to give compound **(I-A)** or **(I-B)** respectively.

2. Process according to Claim 1, wherein R¹, R², R³, R⁴, R¹', R²', R³', R⁴' are each independently a C₁-C₄-alkyl radical,
wherein R⁹, R¹⁰, R⁹', R¹⁰' are each independently selected from the group consisting of hydrogen, C₁-C₄-alkyl radical,
wherein R⁵, R⁶, R⁷, R⁷' are each independently selected from the group consisting of hydrogen, alkyl, where alkyl may be interrupted by one or more divalent radicals not directly adjacent to one another selected from the group consisting of oxygen, imino, and where, in addition, R⁵ and R⁶ may form an alkylene group which may be interrupted by one or more divalent radicals not directly adjacent to one another selected from the group consisting of oxygen, imino,
wherein R⁸ is an alkylene group which may be substituted by one or more radicals selected from the group consisting of alkyl, hydroxy, nitro, carboxyl, carboxyl ester, carboxyl amide, sulfonate, sulfonic acid ester, sulfonamide, halogen, and which may be interrupted by one or more divalent radicals not directly adjacent to one another selected from the group consisting of oxygen, imino,
where m = 1.

3. Process according to Claim 2, wherein R¹, R², R³, R⁴, R¹', R²', R³', R⁴', R⁵, R⁶, R⁷ are in each case methyl, R⁹, R¹⁰, R⁹', R¹⁰' are in each case hydrogen, R⁸ is selected from the group consisting of ethylene, butylene, hexamethylene, and m = 1.

4. Process according to any of Claims 1 to 3, wherein the melt **S** of at least one compound of the formulae **(II-A)** and **(II-B)** is used as a mixture **M** with at least one aprotic organic solvent, wherein the total amount of all aprotic organic solvents used is not sufficient to dissolve the melt **S** therein.

5. Process according to Claim 4, wherein the amount of all aprotic organic solvents contained in the mixture **M** is adjusted so that at least 50 mol% of all compounds of the formulae **(II-A)** and **(II-B)** contained in the mixture **M** are present as melt **S.**

6. Process according to any of Claims 1 to 5, wherein the at least one compound of the formula **(I-A)** or **(I-B)** obtained precipitates at least partially as a precipitate from the melt **S,** and is at least partially separated off as a fraction **F** from the melt **S.**

7. Process according to Claim 6, wherein the fraction **F** also comprises at least one compound of the formula **(II-A)** or **(II-B),** which is separated at least proportionally into product of the formula **(I-A)** or **(I-B)** and at least one reactant of the formula **(II-A)** or **(II-B),** and the at least one compound of the formula **(II-A)** or **(II-B)** separated off from this fraction is recycled again to the melt **S.**

8. Process according to any of Claims 1 to 7, which is carried out at a temperature of 0°C to 200°C and a pressure of 0.5 bar to 10 bar.

9. Process according to any of Claims 1 to 8, which is carried out continuously.

## Revendications

1. Procédé de préparation d'au moins un composé de Formule **(I-A)** ou **(I-B)** 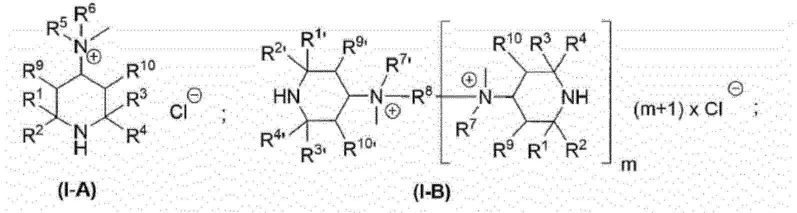
dans lequel R¹, R², R³, R⁴, R¹', R²', R³', R⁴' représentent chacun indépendamment des autres un radical alkyle en C₁-C₆,
dans lequel R⁹, R¹⁰, R⁹', R¹⁰' sont choisis chacun indépendamment des autres dans le groupe consistant en un hydrogène, un radical alkyle en C₁-C₆,
dans lequel R⁵, R⁶, R⁷, R⁷' sont chacun indépendamment des autres choisis dans le groupe consistant en un hydrogène, un alkyle, un cycloalkyle, un aryle, un aralkyle, un hétérocyclyle, les radicaux alkyle, cycloalkyle, aryle, aralkyle, hétérocyclyle pouvant être substitués par un ou plusieurs radicaux choisis dans le groupe consistant en les radicaux alcoxy, hydroxy, carboxyle, ester d'acide carboxylique, carboxamide, sulfonate, ester d'acide sulfonique, sulfonamide, halogène, par ailleurs l'alkyle pouvant être interrompu par un ou plusieurs radicaux divalents, non directement voisins l'un de l'autre, choisis dans le groupe consistant en un oxygène, un imino, et par ailleurs R⁵ et R⁶ pouvant former ensemble un groupe alkylène, qui peut être interrompu par un ou plusieurs radicaux divalents, non directement voisins l'un de l'autre, choisis dans le groupe consistant en un oxygène, un imino,
dans lequel R⁸ représente un groupe pontant organique (m+1)-valent, qui peut être substitué par un ou plusieurs radicaux choisis dans le groupe consistant en les radicaux alkyle, hydroxy, nitro, carboxyle, ester d'acide carboxylique, carboxamide, sulfonate, ester d'acide sulfonique, sulfonamide, halogéno, et qui peut être interrompu par un ou plusieurs radicaux divalents, non directement voisins l'un de l'autre, choisis dans le groupe consistant en un oxygène, un imino,
dans lequel m représente un entier de 1 à 5, **caractérisé en ce qu'**on envoie du chlorure de méthyle gazeux dans une masse fondue **S** d'au moins un composé de Formule **(II-A)** ou **(II-B)** 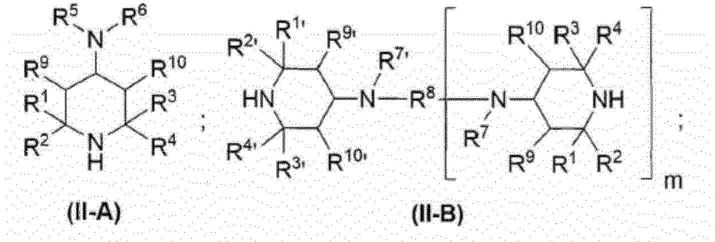
de façon que le chlorure de méthyle et l'au moins un composé de Formule **(II-A)** ou **(II-B)** réagissent pour donner le composé respectivement **(I-A)** et **(I-B).**

2. Procédé selon la revendication 1, dans lequel R¹, R², R³, R⁴, R¹', R²', R³', R⁴' représentent chacun indépendamment des autres un radical alkyle en C₁-C₄, dans lequel R⁹, R¹⁰, R⁹', R¹⁰' sont chacun indépendamment des autres choisis dans le groupe consistant en un hydrogène, un radical alkyle en C₁-C₄,
dans lequel R⁵, R⁶, R⁷, R⁷' sont choisis chacun indépendamment des autres dans le groupe consistant en un hydrogène, un alkyle, l'alkyle pouvant être interrompu par un ou plusieurs radicaux divalents, non directement voisins l'un de l'autre, choisis dans le groupe consistant en un oxygène, un imino, et dans lequel par ailleurs R⁵ et R⁶ peuvent former un groupe alkylène, qui peut être interrompu par un ou plusieurs radicaux divalents, non directement voisins l'un de l'autre, choisis dans le groupe consistant en un oxygène, un imino,
dans lequel R⁸ représente un groupe alkylène, qui peut être substitué par un ou plusieurs radicaux choisis dans le groupe consistant en les radicaux alkyle, hydroxy, nitro, carboxyle, ester d'acide carboxylique, carboxamide, sulfonate, ester d'acide sulfonique, sulfonamide, halogéno, et qui peut être interrompu par un ou plusieurs radicaux divalents, non directement voisins l'un de l'autre, choisis dans le groupe consistant en un oxygène, un imino,
dans lequel m = 1.

3. Procédé selon la revendication 2, dans lequel R¹, R², R³, R⁴, R¹', R²', R³', R⁴', R⁵, R⁶, R⁷ représentent chacun un méthyle, R⁹, R¹⁰, R⁹', R¹⁰' représentent chacun un hydrogène, R⁸ est choisi dans le groupe consistant en un éthylène, un butylène, un hexaméthylène, et m = 1.

4. Procédé selon l'une des revendications 1 à 3, dans lequel la masse fondue **S** d'au moins un composé de Formules **(II-A)** et **(II-B)** est utilisée sous forme d'un mélange **M** avec au moins un solvant organique aprotique, la quantité totale de l'ensemble des solvants organiques aprotiques utilisés ne suffisant pas pour y dissoudre la masse fondue **S.**

5. Procédé selon la revendication 4, dans lequel la quantité de la totalité des solvants organiques aprotiques englobés par le mélange **M** est ajustée de façon qu'au moins 50 % en moles de l'ensemble des composés englobés dans le mélange **M** de Formules **(II-A)** et **(II-B)** se présentent sous forme de la masse fondue **S.**

6. Procédé selon l'une des revendications 1 à 5, dans lequel l'au moins un composé obtenu de Formule **(I-A)** ou **(I-B)** précipite au moins partiellement dans la masse fondue **S** sous forme d'un dépôt, et est séparé au moins partiellement de la masse fondue **S** sous forme d'une fraction **F.**

7. Procédé selon la revendication 6, dans lequel la fraction **F** comprend elle aussi au moins un composé de Formule **(II-A)** ou **(II-B),** au moins en partie dans le produit de Formule **(I-A)** ou **(I-B),** et au moins un réactif de Formule **(II-A)** ou **(II-B)** est séparé, et l'au moins un composé de Formule **(II-A)** ou **(II-B),** séparé de cette fraction, est renvoyé dans la masse fondue **S.**

8. Procédé selon l'une des revendications 1 à 7, qui est mis en œuvre à une température de 0 °C à 200 °C et sous une pression de 0,5 bar à 10 bar.

9. Procédé selon l'une des revendications 1 à 8, qui est mis en œuvre en continu.
